# EUROPEAN PATENT APPLICATION

(11) **EP 3 456 370 A1**
(43) Date of publication of application: **20.03.2019**
(21) Application number: 18193981.0
(22) Date of filing: 12.09.2018
(51) Int. Cl.: A61M 16/06, A61G 12/00, B65H 75/00, H02G 7/00, A61M 16/08

(54) **DEVICE FOR TRANSPORTING A GAS, SUCH AS OXYGEN**

(30) Priority: 14.09.2017 NL 2019539
(71) Applicant: Oxyrunner B.V., 4206 AP Gorinchem (NL)
(72) Inventor: De Kruijk, Johannes Wouter, 4206 AP Gorinchem (NL)
(74) Representative: 't Jong, Bastiaan Jacob

(57) **Abstract**

The invention relates to a device (1) for transporting a gas, such as oxygen, comprising:
- a tube (4), connected with a first outer end to a gas source (5)
wherein:
- the device comprises guide means (6); and
- the tube is mounted displaceably in a guiding direction on the guide means at at least one mounting point (9) along the guide means.

## Description

The invention relates to a device for transporting a gas, such as oxygen, comprising:
- a tube, connected with a first outer end to a gas source.

In the case of various pulmonary diseases a patient may be insufficiently able, for instance due to damage to the airways or lungs such as alveoli, to transport enough oxygen to the blood without auxiliary means. This is for instance the case with the medical indication known by the acronym COPD (chronic obstructive pulmonary disease).

For this reason it is known to supply oxygen to such patients, for instance via the nose, via an oxygen machine. For this purpose a tube is arranged between a gas source, in this case an oxygen source, and the patient, which tube is transported along the floor.

Such existing devices have drawbacks. The transport along the floor entails a considerable risk of the patient tripping over the tube, resulting in injury, or being obstructed by the tube. As the floor area of the house in which such a device is being used increases, this drawback will become increasingly more prominent. The tube can furthermore wear and begin to leak relatively quickly as a result of being transported along the floor.

It is now an object of the invention to reduce or even prevent the above stated problems.

This object is achieved with a device according to the preamble, wherein:
- the device comprises guide means; and
- the tube is mounted displaceably in a guiding direction on the guide means at at least one mounting point along the guide means.

Provided in the device according to the invention are guide means, on which the tube is displaceably mounted at at least one mounting point. Such guide means can be fixed at various locations so that it is no longer necessary to drag the tube along the floor, which prevents the risk of tripping and wear of the tube.

From the most distal mounting point on the guide relative to the gas source, such as the most distal carriage (if applicable), the tube preferably runs to the ankles of the user and then on to the body cavity, such as the nasal cavity. A great freedom of movement is in this way achieved, while the risk of tripping over the tube still remains limited because this tube is not in contact with the floor.

In a first preferred embodiment of the device according to the invention the guide means comprise a guide rail and the device comprises at least one carriage mounted displaceably along the guide rail on the guide means, wherein the tube is mounted on the at least one carriage at the at least one mounting point.

Mounting the tube on a carriage which is displaceable along and over the guide rail provides the option of mounting the tube on the guide means, without the tube having to be modified for this purpose. In that case the mounting of the tube on the guide means is thus done via the mounting of the carriage on the guide means.

There are preferably a plurality of carriages because this further increases the option of storing a relatively long tube over a short distance at the moment that the distance between the carriages is relatively small.

An example of a suitable guide rail is a rail for curtains, as is currently known.

Because the tube is mounted on the carriage, the tube is prevented from sliding relative to the carriage.

In a second preferred embodiment of the device according to the invention at least one of the carriages comprises a support surface bent toward the guide means parallel to the guiding direction, and the tube extends over the support surface.

It is advantageous to incorporate in the carriage a support surface which is bent toward the guide means and which preferably runs toward the guide means, and runs away from the guide means on the other side, because an easy mounting of the tube on the carriage can be achieved in this way without the tube becoming kinked.

In a third preferred embodiment of the device according to the invention the device further comprises connecting means, such as a cord, these connecting means being coupled to one of the at least one mounting point and further being coupled to another of the at least one mounting point or a further mounting point such as a wall.

Coupling successive mounting points, such as for instance successive carriages, to each other and/or to a further mounting point such as a wall further increases the reliability of the movement of the mounting points relative to each other.

In a fourth preferred embodiment of the device according to the invention the length of the connecting means between two successive mounting points is shorter than the length of the tube.

In this way the tube is prevented from being pulled tight at all times, since the tube hangs loosely at the moment that the connecting means are pulled tight.

In a fifth preferred embodiment of the device according to the invention the device further comprises a handle coupled to one of the mounting points.

A handle, such as for instance a stick or rope, enables the mounting points to be displaced. The handle is here preferably coupled and mounted on the mounting point or carriage furthest removed from the gas source and closest to the user of the device in order to increase the convenience of use. The handle is preferably not coupled to the user so as to reduce the chance of the user hurting him or herself on the handle.

In a sixth preferred embodiment of the device according to the invention the device further comprises mounting means for mounting the guide means on a ceiling.

By mounting the device on a ceiling using mounting means the tube remains for the greater part at a great distance from the floor, and the chance of tripping and premature wear is reduced.

In a seventh preferred embodiment of the device according to the invention the device further comprises a support frame, wherein the guide means support on the support frame.

Having the guide means rest on a support frame creates the option of allowing the device to run in places where no ceiling is present, or where mounting on a ceiling is undesirable, such as for instance in a garden.

In an eighth preferred embodiment of the device according to the invention the support frame comprises at least one and preferably a plurality of uprights placed at a mutual distance.

The use of uprights placed at a mutual distance is an inexpensive embodiment of a support frame which can be easily installed.

In a ninth preferred embodiment of the device according to the invention coupling means are arranged on a second outer end of the tube for the purpose of coupling the tube to or in a body cavity, such as a human nasal cavity.

With these coupling means it is possible to place or clamp the tube in a body cavity, such as the human nasal cavity.

These and other aspects of the invention are elucidated with reference to the accompanying drawing.

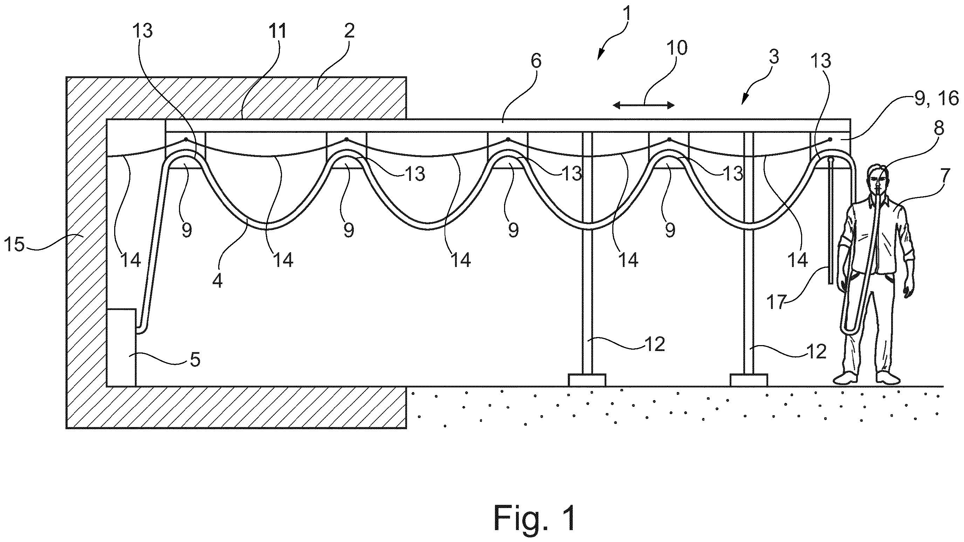

Figure 1 shows a side view of a device according to the invention.

Figure 1 shows a device 1 which is placed partially in a house 2 and partially in a garden 3 adjoining the house 2. A tube 4 runs from a gas source 5 along a guide rail 6 and to a patient 7, where it is placed via coupling means (not shown) in the nose 8 of patient 7. Tube 4 is here displaceable reciprocally along guide rail 6 in direction 10 by means of carriages 9. In so far as guide rail 6 runs inside house 2, the guide rail is mounted on ceiling 11 of house 2, and in so far as it runs through garden 3 it is supported by uprights 12 placed at a mutual distance. Each of the carriages 9 comprises a support surface 13 bent toward guide rail 6. Running between carriages 9 are coupling means in the form of a cord 14, which are further attached to wall 15 of house 2. The length of cord 14 is here shorter than the length of the tube 4 between carriages 9, so that tube 4 always hangs loosely between carriages 9. A handle 17 is further arranged on the carriage 16 furthest removed from gas source 5. From carriage 16, tube 4 first runs to the ankles of patient 7 and then on to the nose 8 of patient 7.

## Claims

1. Device for transporting a gas, such as oxygen, comprising:
- a tube, connected with a first outer end to a gas source
**characterized in that**
- the device comprises guide means; and
- the tube is mounted displaceably in a guiding direction on the guide means at at least one mounting point along the guide means.

2. Device as claimed in claim 1, wherein the guide means comprise a guide rail and wherein the device comprises at least one carriage mounted displaceably along the guide rail on the guide means, wherein the tube is mounted on the at least one carriage at the at least one mounting point.

3. Device as claimed in claim 2, wherein at least one of the carriages comprises a support surface bent toward the guide means parallel to the guiding direction, and wherein the tube extends over the support surface.

4. Device as claimed in claim 1, 2 or 3, further comprising connecting means, such as a cord, these connecting means being coupled to one of the at least one mounting point and further being coupled to another of the at least one mounting point or a further mounting point such as a wall.

5. Device as claimed in claim 4, wherein the length of the connecting means between two successive mounting points is shorter than the length of the tube.

6. Device as claimed in any one of the foregoing claims, further comprising a handle coupled to one of the mounting points.

7. Device as claimed in any one of the foregoing claims, further comprising mounting means for mounting the guide means on a ceiling.

8. Device as claimed in any one of the foregoing claims, wherein the device further comprises a support frame, wherein the guide means support on the support frame.

9. Device as claimed in claim 8, wherein the support frame comprises at least one and preferably a plurality of uprights placed at a mutual distance.

10. Device as claimed in any one of the foregoing claims, wherein coupling means are arranged on a second outer end of the tube for the purpose of coupling the tube to or in a body cavity, such as a human nasal cavity.
